# EUROPEAN PATENT APPLICATION

(11) **EP 4 485 478 A1**
(43) Date of publication of application: **01.01.2025**
(21) Application number: 23181949.1
(22) Date of filing: 28.06.2023
(51) Int. Cl.: G16H 50/50

(54) **INTERACTIVE AVATAR FOR MEDICAL DATA RETRIEVAL**

(71) Applicant: Siemens Healthineers AG, 91301 Forchheim (DE)
(72) Inventor: CHAGANTI, Shikha, Princeton, 05480 (US); YU, Daphne, Yardley, 19067 (US)
(74) Representative: Schwarz, Claudia

(57) **Abstract**

A method for generating an interactive avatar of a patient for medical data retrieval comprises receiving a graphical representation of a human body. A medical knowledge dataset is received (S104) in relation to the patient. An anatomical location algorithm is executed for linking (S106) medical information comprised in the medical knowledge dataset to one or more associated locations of the graphical representation for obtaining (S108) an avatar (402) of the patient. An interactive avatar (402) is generated (S110) by supplementing the avatar (402) of the patient with a user interaction tool.

## Description

The present invention relates to a technique for generating an interactive avatar of a patient for medical data retrieval and optional guidance, in particular comprising a method, a computing device, a system comprising the computing device, and a computer program product.

Conventionally, medical knowledge comprises general knowledge as well as patient-specific knowledge. Additionally, medical knowledge may be classified in terms of imaging data and non-imaging data.

For imaging data, there exist research papers and products to segment organs from medical imaging. Likewise, for non-imaging data there exist research papers for extraction of clinical and anatomical entities from electronic health records (EHRs) and medical texts. However, there is no solution which allows to integrate these extracted entities and organize them for quick, anatomically correct and comprehensive retrieval and/or guidance.

It is therefore an object of the present invention to provide a solution for quick, anatomically correct, locally and/or causally connected, and/or comprehensive retrieval of medical data. Alternatively or in addition, it is an object of the present invention to provide a technique for comprehensive and clear guidance during the course of a medical treatment and/or consultation.

This object is solved by a method for generating an interactive avatar of a patient for medical data retrieval, by a computing device, by a system comprising the computing device, by a computer program (and/or computer program product), and by a computer-readable storage medium according to the appended independent claims. Advantageous aspects, features and embodiments are described in the dependent claims and in the following description together with advantages.

In the following, the solution according to the invention is described with respect to the claimed method for generating an interactive avatar of a patient for medical data retrieval as well as with respect to the claimed computing device and the claimed system comprising the computing device. Features, advantages or alternative embodiments herein can be assigned to the other claimed objects (e.g., the computer program or a computer program product), and vice versa. In other words, claims for the computing device, and/or the system comprising the computing device, can be improved with features described or claimed in the context of the method. In this case, the functional features of the method are embodied by structural units (e.g., interface and/or modules) of the computing device, and/or the system, and vice versa, respectively.

As to a method aspect, a method for generating an interactive avatar of a patient for medical data retrieval is provided. The method comprises a step of receiving a graphical representation of a human body (e.g., a non-patient specific human body, or corresponding to the, in particular outer shape of the patient). The method further comprises a step of receiving at least one (in particular textual, image and/or anatomical) medical knowledge dataset in relation to the patient from a medical knowledge database. The method further comprises a step of executing an anatomical location algorithm for linking medical information comprised in the received at least one medical knowledge dataset to one or more associated locations of the received graphical representation of the patient
for obtaining an avatar of the patient.

The method still further comprises a step of generating an interactive avatar of the patient by supplementing the obtained avatar of the patient with a user interaction tool (e.g., comprising a user interface, UI, and/or a graphical user interface, GUI).

The method steps may be performed in a different order from the one presented above. E.g., the step of receiving at least one medical knowledge dataset in relation to the patient may precede the step of receiving the graphical representation of the human body.

Alternatively or in addition, the steps of receiving the graphical representation of the human body and/or of receiving at least one medical knowledge dataset in relation to the patient may (e.g., at first) comprise receiving (e.g., only) non-patient-specific information.

Further alternatively or in addition, one or more method steps may be performed simultaneously. E.g., the step of receiving at least one medical knowledge dataset in relation to the patient may be performed simultaneously with the step of receiving the graphical representation of the human body. Alternatively or in addition, the generating of the interactive avatar by supplementing a user interaction tool may be performed simultaneously with executing the anatomical location algorithm. Further alternatively or in addition, the obtaining of the avatar of the patient may (e.g., immediately) comprise supplementing the avatar of the patient with a user interaction tool.

By the inventive technique, medical data are provided for retrieval in a comprehensive and locally connected, and/or causally connected manner, allowing for faster and more pertinent retrieval of the medical data potentially relevant to one or more medical symptoms and/or treatments. Thereby, an improved medical diagnosis and/or an improved treatment plan (also denoted as therapy plan) can be achieved, and/or misdiagnoses and/or selecting inappropriate treatment measures can be avoided. Alternatively or in addition, a (e.g., optical and/or acoustic) guidance of a medical practitioner during a consultation and/or during an intervention (also denoted as treatment, procedure, and/or surgical procedure) may be improved. Thereby, a patient outcome may be improved.

The patient may also be denoted as individual human body. Alternatively or in addition, the medical practitioner may generally also be denoted as physician, and/or according to a specialization, e.g., as surgeon.

The avatar of the patient may also be denoted as digital twin avatar of the patient (briefly also: digital twin avatar). Alternatively or in addition, the (e.g., interactive) avatar of the patient may also be denoted as (e.g., interactive) patient-specific avatar, (e.g., interactive) digital twin avatar of the patient, (e.g., interactive) patient-specific digital twin avatar, (e.g., interactive) individual digital twin avatar, and/or as (e.g., interactive) individual avatar.

The graphical representation may comprise a (e.g., three-dimensional, 3D) mesh representation. Alternatively or in addition, the graphical representation may comprise a surface representation of the (e.g., naked) human body.

The graphical representation may be non-patient-specific (e.g., corresponding to a class of human bodies to which the patient belongs, such as grown-ups of a specified biological sex) or patient-specific. The patient-specific graphical representation may in particular be obtained by means of a camera (e.g., for taking at least one photograph, and/or a video sequence, of the patient).

In some embodiments, the graphical representation may comprise anatomical structures, in particular organ systems, and/or organs. Alternatively or in addition, in some embodiments the graphical representation may comprise a hierarchical structure, e.g., from the entire human body to organ structures, to organs and to increasingly finer granularity of parts of a human body. E.g., by using the user interaction tool, a zoom-in or zoom-out between different levels of the hierarchical structure may be performed. Further alternatively or in addition, in some embodiments the graphical representation may comprise a segmentation of a (e.g., entire) human body, e.g., representative of a class of human bodies.

A class of human bodies (e.g., in case of a non-patient-specific graphical representation) may comprise a classification in terms of the presence and/or development state of body parts, anatomical structures and/or organs. Alternatively or in addition, the class of human bodies may comprise a classification in terms of biological sex (e.g., men and women may correspond to different classes), and/or a classification in terms of a development state (and/or age range) of the human body (e.g., infants, toddlers, children, adolescents, and/or grown-ups may correspond to different classes).

The medical knowledge dataset may comprise a textual medical knowledge dataset and/or an image medical knowledge dataset. Alternatively or in addition, any one of the medical knowledge dataset, the textual medical knowledge dataset, and/or the image medical knowledge dataset may comprise non-patient-specific data (e.g., data for a class of human bodies) and/or patient-specific data.

The (e.g., non-patient-specific) textual medical knowledge dataset (and/or the textual medical information) may comprise a medical ontology database (and/or medical ontology). Alternatively or in addition, the textual medical knowledge dataset (and/or the textual medical information) may comprise, and/or may be based on, the systematized nomenclature of medicine (SNOMED) and/or the foundational model of anatomy (FMA) .

The (e.g., non-patient-specific) textual medical knowledge database may comprise a plurality of textual medical knowledge datasets, in particular each associated with a different class of human bodies. Alternatively or in addition, a (e.g., non-patient-specific) textual medical knowledge dataset may comprise information on a specific anatomical structure, a member of the hierarchical structure (e.g., an organ system or an organ), and/or illness symptoms.

The (e.g., non-patient-specific) image medical knowledge database may comprise a plurality of image medical knowledge datasets, in particular each associated with a different class of human bodies. Alternatively or in addition, a (e.g., non-patient-specific) image medical knowledge dataset may comprise an (e.g., schematic) image of a specific anatomical structure, and/or of a member of the hierarchical structure (e.g., an organ system or an organ.

The graphical representation, and/or the medical knowledge dataset, may comprise textual labels, denominations, and/or annotations of the anatomical structures, organ structures, organs, and/or any hierarchical structure of the (e.g., class of) human bodies.

The anatomical location algorithm may also be denoted as anatomical location algorithm.

The anatomical location algorithm may be configured for associating (e.g., textual and/or image) medical information with one or more locations of the graphical representation, in particular by means of the textual labels, denominations, and/or annotations comprised in the graphical representation.

The textual medical information of the at least one textual medical knowledge dataset, in particular in association with the one or more locations of the graphical representation, may also be denoted as medical knowledge corresponding to an anatomy.

The associating of the textual medical information with one or more locations of the graphical representation may be based on comparing the textual labels, denominations, and/or annotations of the graphical representation with the textual medical information comprised in the one or more textual medical knowledge datasets.

The (e.g., at least one) patient-specific medical knowledge dataset in relation to the patient may also be denoted as (e.g., at least one) patient-specific medical information dataset.

The at least one patient-specific medical knowledge dataset (and/or patient-specific medical information dataset) may comprise medical imaging data of the patient (and/or individual human body). E.g., the medical imaging data may comprise a segmentation of one or more anatomical structures (in particular organs) of the patient, in particular with annotations and/or textual labels.

The medical imaging data may be acquired using a medical scanner with an associated medical imaging modality. The medical imaging modality may comprise magnetic resonance imaging (MRI), computed tomography (CT), ultrasound (US), radiography (also denoted as X-ray imaging), positron emission tomography (PET), and/or single-photon emission computed tomography (SPECT) .

Alternatively or in addition, the (e.g., at least one) patient-specific medical knowledge dataset (and/or patient-specific medical information dataset) may comprise (e.g. patient-specific) textual information (e.g., preferably comprising numerical information in relation to measured patient-specific values and/or vital signs), e.g., an electronic health record (EHR), a medical report (e.g., in relation to a medical consultation and/or surgical procedure), laboratory results (e.g., in relation to a body fluid analysis, in particular a blood and/or urine analysis), measured vital signs (e.g., blood pressure, heart rate, blood sugar level, electrocardiogram, ECG, body temperature, and/or respiratory rate), measured body shape indices (e.g., height, weight, and/or body mass index, BMI), and/or prescribed medications (e.g., comprising dosage and/or intake instructions).

The textual information may be (e.g., directly) received from a sensor (e.g., comprised in a wearable device). Alternatively or in addition, the textual information may be received by means of a microphone (e.g., for dictating a medical report, and/or laboratory results), and/or by means of an input using a UI, e.g., a keyboard and/or mouse, and/or a GUI (e.g., a screen of a tablet computer).

In some embodiments, at first a non-patient-specific (als denoted as class-specific avatar of a class of human bodies, to which the patient belongs, may be obtained as a result of the linking, which may be adapted to the avatar of the patient (also denoted as patient-specific avatar).

The adapting (and/or obtaining, and/or creating an avatar of the patient) may comprise modifying, based on at least one received patient-specific medical knowledge dataset.

Alternatively or in addition, in some embodiments the adapting of the class-specific avatar to the (e.g., interactive) avatar of the patient may comprise modifying the graphical representation (e.g., based on medical imaging data of the patient, and/or based on an indication of a surgical modification, e.g., an amputation, comprised in a medical report).

Alternatively or in addition, the adapting of the class-specific avatar to the (e.g., interactive) avatar of the patient may comprise modifying the (e.g., textual) information in association with one or more locations of the graphical representation based on the patient-specific (e.g., textual) information (e.g., in relation to an anatomical structure and/or an organ, in particular comprised in a medical report, and/or to which laboratory results correspond).

The (e.g., interactive) avatar of the patient may also be denoted as patient-specific (e.g., interactive) avatar. Alternatively or in addition the (e.g., interactive) avatar of the patient may also be denoted as (in particular anatomically indexed) patient data model.

The step of linking textual medical information of the at least one (e.g., textual and/or image) medical knowledge dataset with one or more locations of the graphical representation to obtain the avatar of the patient may also be denoted as linking, and/or populating, the (in particular anatomically indexed) patient data model (briefly also: linking, and/or populating, data points).

A data point may comprise a dataset (e.g., the one or more, in particular textual and/or image, medical knowledge datasets, which may each be patient-specific or non-patient-specific).

The non-patient-specific graphical representation of the human body, the (e.g., textual and/or image) medical information of the at least one (e.g., textual and/or image) medical knowledge dataset, and/or the class-specific avatar may also be denoted as canonical representation. Alternatively or in addition, the patient-specific at least one medical knowledge dataset, and/or the (e.g., interactive) avatar of the patient may also be denoted as personalized representation.

The supplementing of the user interaction tool may comprise furnishing the avatar of the patient with a navigation tool, e.g., by means of a UI, in particular a GUI.

The method may further comprise a step of outputting the generated interactive avatar of the patient, in particular on a GUI.

The interactive avatar of the patient (and/or the patient-specific interactive avatar) may be output on a display of a computing device, a tablet computer, and/or a smart phone. Alternatively or in addition, the output of the interactive avatar of the patient may comprise a projection (e.g., onto a screen, wall, and/or the patient).

The graphical representation may comprise a (in particular 3D) mesh representation. Alternatively or in addition, the graphical representation may be algorithmically generated.

The graphical representation may be non-patient-specific. Alternatively, the graphical representation may be patient-specific. The patient-specific graphical representation may be generated based on input data indicative of a scan of the patient's outer body shape (and/or body surface), in particular based on at least one photograph and/or on a sequence of images taken by a (e.g., video) camera.

The graphical representation, the at least one (e.g., textual and/or image) medical knowledge dataset, the class-specific avatar, and/or the (in particular interactive) avatar of the patient may be stored in a local database. Alternatively or in addition, the graphical representation, the at least one (e.g., textual and/or image) medical knowledge dataset, the class-specific avatar, and/or the (in particular interactive) avatar of the patient may be stored in a computing cloud.

Further alternatively or in addition, the storage of the graphical representation, the at least one (e.g., textual and/or image) medical knowledge dataset, the class-specific avatar, and/or the (in particular interactive) avatar of the patient may be distributed over different storage (and/or hardware) entities. In particular, the graphical representation and the medical knowledge dataset may stem from different storage origins and/or are stored in different storage entities and/or are received from different storage entities.

The local database may be stored on the computing device, tablet computer, and/or smart phone.

The graphical representation (e.g., for a class of human bodies, and/or non-patient-specific) may comprise a biological sex type, and/or a development state, in particular comprising infant, adolescent and/or grown-up.

The biological sex type may comprise male and/or female. Alternatively or in addition, the development state may correspond to a development of parts of the body, anatomical structures, and/or organs (e.g., comprising no teeth and/or deciduous teeth for an infant, and/or molar teeth and/or wisdom teeth for an adult; comprising an age-typical ratio of different bone structures, - in particular sensory - organs, and/or limbs; and/or comprising a development state of biological sex-specific genitalia according to an age of the patient) .

The (in particular patient-specific) received at least one medical knowledge dataset in relation to the patient may comprise medical imaging data of the patient, in particular received from a medical scanner. Optionally, the medical imaging data may comprise annotations and/or textual labels.

The medical scanner may comprise an MRI scanner, a CT scanner, a US scanner, an X-ray scanner, a PET scanner, and/or a SPECT scanner. Alternatively or in addition, the medical imaging data may comprise one or more images of one or more anatomical structures (e.g., organs), one or more segmentations of one or more anatomical structures (e.g., organs), and/or one or more images of a fracture (e.g., of at least one bone).

The annotations may comprise a (e.g., organ) segmentation, and/or textual labels.

Alternatively or in addition, the (in particular patient-specific) received at least one medical knowledge dataset in relation to the patient may comprise non-imaging medical data of the patient.

The non-imaging medical data of the patient (and/or of the patient) may comprise an electronic health record (EHR), a medical report, laboratory results, and/or vital signs, in particular acquired by means of a wearable device. The wearable device (e.g., comprising a smart watch) may measure (and/or store, and/or transmit), e.g., vital signs such as blood pressure, heart rate, blood sugar level, and/or an electrocardiogram (ECG).

The medical data acquired by a wearable device may also be denoted as wearable data.

The inventive technique may make use of data object types denoted as medical knowledge (e.g., comprising the graphical representation, one or more medical ontologies, and/or medical publications), (in particular patient-specific) non-imaging data (e.g., comprising medical reports, laboratory results, and/or vital signs), and/or (in particular patient-specific) imaging data (e.g., comprising MRI scans, CT scans, X-ray scans, and/or associated segmentations).

The linking of the (e.g., textual and/or image) medical information comprised in the received at least one medical knowledge dataset to the one or more associated locations of the received graphical representation may be performed by means of natural language processing (NLP) and/or by means of a text matching algorithm to a medical ontology database.

The NLP, and/or the text matching algorithm, may be used to match text labels (and/or annotations) of imaging data, in particular segmentations, to textual medical information.

The linking of the textual medical information comprised in the received at least one textual medical knowledge dataset to the one or more associated locations of the received graphical representation may be performed by means of an artificial intelligence (AI).

The AI may comprise one or more (in particular trained) neural networks, a machine learning algorithm, and/or reinforcement learning.

The graphical representation and/or the (e.g., textual and/or image) medical information comprised in the received at least one medical knowledge dataset may be hierarchically organized, in particular comprising the entire human body (e.g., of the patient), organ systems, organs and suborganic structures as subsequent hierarchical layers.

The entire human body (e.g., comprising a biological sex type as the class) may correspond to the highest hierarchical layer. Alternatively or in addition, one or more organ systems (e.g., the cardiovascular system, the respiratory system, and/or the skeletal system) may correspond to a layer (e.g., the next-to-highest layer) below the entire human body. Further alternatively or in addition, one or more organs (e.g., comprising the lung lobes, right and left lungs, and/or airway tree) may correspond to a layer below the organ system layer (e.g., below the respiratory system layer).

The arrangement of successively lower hierarchical layers may correspond to a successive refinement of an anatomical structure.

The hierarchical organization can enable an efficient retrieval of medical data by zooming-in on a particular location, and/or a particular functionality (e.g., in relation to an organ system). The efficiency may refer to computing resources (e.g., in terms of processor and/or memory usage), and/or a time for retrieval.

The (e.g., textual and/or image) medical information comprised in the received at least one medical knowledge dataset may be represented by a graph data structure. Nodes of the graph data structure may comprise clinical concepts. Edges between nodes of the graph data structure may comprise relationships between the clinical concepts.

The (e.g., textual and/or image, in particular non-patient-specific) medical information comprised in the received at least one medical knowledge dataset may comprise a (or may also be denoted as) clinical ontology database (briefly also: clinical ontology).

The clinical concept (in particular represented by a node of the graph data structure) may, e.g., comprise an anatomical structure such as an organ system, and/or an organ. Alternatively or in addition, a relationship (in particular represented by an edge between nodes of the graph data structure) may comprise a hierarchy, a disease type, an anatomical location, and/or one or more (e.g., healthy, unhealthy, and/or patient-specific) laboratory values and/or vital signs.

The method may further comprise a step of receiving, by means of the interactive user tool, a request for medical data retrieval. Alternatively or in addition, the method may further comprise a step of outputting medical data, in particular according to a received request. Further alternatively or in addition, the method may further comprise a step of receiving at least one further medical information dataset in relation to the patient for updating the interactive avatar of the patient.

The method of generating an interactive avatar of a patient for medical data retrieval may comprise a use of the method for data retrieval by means of the interactive avatar of a human body.

The received request for medical data retrieval may also be denoted as search request, and/or as (in particular multi-) search.

The received request for medical data retrieval may comprise a navigation. The navigation may relate to the hierarchical organization, and/or to nodes (and/or edges) of the graph data structure. E.g., the navigation may comprise a zooming-in on successively refined anatomical structures (and/or lower hierarchical layers). Alternatively or in addition, the navigation according to the graph data structure may comprise uncovering the relationship between successively refined clinical concepts.

The output of the medical data, in particular according to the received request, may comprise an infographic.

The receiving of the at least one further medical information dataset in relation to the patient may comprise receiving further medical imaging data, and/or further non-imaging medical data.

The method may be used (in particular for optical guidance) during an interventional procedure and/or during a consultation.

The interventional procedure may also be denoted as surgical procedure. Alternatively or in addition, the interventional procedure may comprise a medical treatment of the patient.

As to a device aspect, a computing device for generating an interactive avatar of a patient for medical data retrieval is provided. The computing device comprises a first interface configured for receiving a graphical representation of a human body (e.g., a non-patient specific human body, or corresponding to the, in particular outer shape of the, patient). The computing device further comprises a second interface configured for receiving at least one (e.g., textual and/or image) medical knowledge dataset in relation to the patient from a medical knowledge database. The computing device further comprises a linking module configured for executing an anatomical location algorithm for linking (e.g., textual and/or image) medical information comprised in the received at least one (e.g., textual and/or image) medical knowledge dataset to one or more associated locations of the received graphical representation for obtaining an avatar of the patient.

The computing device still further comprises a generating module configured for generating an interactive avatar of the patient by supplementing the obtained avatar of the patient with a user interaction tool.

The computing device may be further configured to perform any one of the method steps, and/or may comprise any one of the features, disclosed in the context of the method aspect.

As to a system aspect, a system for generating an interactive avatar of a patient for medical data retrieval is provided. The system comprises a graphical representation database configured for storing a graphical representation of a human body. The system further comprises a medical knowledge database configured for storing at least one medical knowledge dataset in relation to the patient. The system further comprises a computing device according to the device aspect. The first interface of the computing device is configured for receiving a graphical representation of a human body from the graphical representation database. The second interface of the computing device is configured for receiving at least one (e.g., textual and/or image) medical knowledge dataset in relation to the patient from the medical knowledge database.

Optionally, the medical knowledge database may comprise medical information in relation to the patient acquired by means of an acquisition device.

The acquisition device may comprise a medical scanner, a sensor, a microphone, and/or a UI, in particular a GUI. Alternatively or in addition, the acquisition device may comprise an interface to an electronic health record (EHR) storing patient-specific medical information.

As to a further aspect, a computer program product is provided comprising program elements which induce a computing device to carry out the steps of the method for generating an interactive avatar of a patient for medical data retrieval according to the method aspect, when the program elements are loaded into a memory of the computing device.

As to a still further aspect, a computer-readable medium is provided on which program elements are stored that can be read and executed by a computing device, in order to perform steps of the method for generating an interactive avatar of a patient for medical data retrieval according to the method aspect, when the program elements are executed by the computing device.

The properties, features and advantages of this invention described above, as well as the manner in which they are achieved, become clearer and more understandable in the light of the following description and embodiments, which will be described in more detail in the context of the drawings.

This following description does not limit the invention on the contained embodiments. Same components or parts can be labeled with the same reference signs in different figures. In general, the figures are not for scale.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

- Fig. 1: is a flow chart of a method for generating an interactive avatar of a patient for medical data retrieval according to a preferred embodiment of the present invention;
- Fig. 2: is an overview of the structure and architecture of a computing device for generating an interactive avatar of a patient for medical data retrieval according to a preferred embodiment of the present invention, which computing device may be configured for performing the method of Fig. 1;
- Fig. 3: schematically indicates a hierarchical organization of anatomical structures;
- Fig. 4: schematically illustrates the hierarchical organization of anatomical structures of Fig. 3 within an avatar according to the inventive concept;
- Fig. 5: schematically illustrates a data object for a patient, based on which an interactive avatar may be generated according to the method of Fig. 1, with the data object comprising general medical knowledge as well as patient-specific data, which may comprise non-imaging data and/or imaging data;
- Fig. 6: schematically illustrates extracting non-imaging data and/or imaging data to assemble the data object of Fig. 5, which may comprise an alternative schematic illustration of performing the steps of the inventive method of Fig. 1; and
- Fig. 7: exemplarily schematically illustrates an interactive avatar of a patient, which may be obtained by means of the method of Fig. 1 and/or by making use of the computing device of Fig. 2.

Any reference signs in the claims should not be construed as limiting the scope.

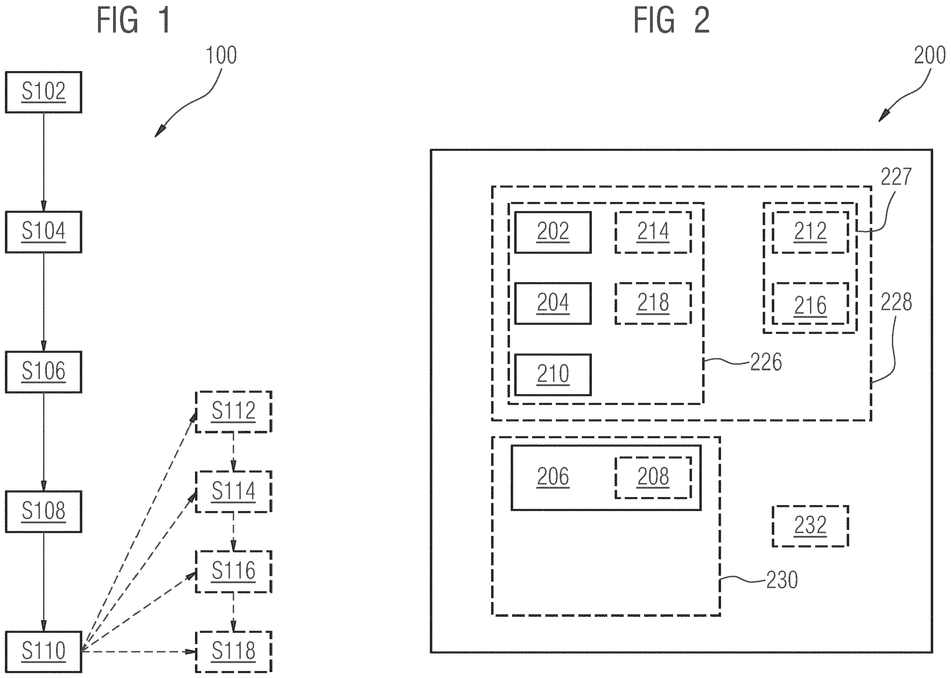

Fig. 1 schematically illustrates an exemplary flowchart of a method for generating an interactive avatar of a patient for medical data retrieval. The method is generally referred to by the reference sign 100.

The method 100 comprises a step S102 of receiving a graphical representation of a human body.. The graphical representation of the human body may be patient-specific or non-patient-specific.

The method 100 further comprises a step S104 of receiving at least one (e.g., textual and/or image) medical knowledge dataset in relation to the patient from a medical knowledge database. The at least one medical knowledge dataset may be patient-specific or non-patient-specific.

The method 100 further comprises a step S106 of executing an anatomical location algorithm for linking (e.g., textual and/or image) medical information comprised in the received S104 at least one (e.g., textual and/or image) medical knowledge dataset to one or more associated locations of the received S102 graphical representation.

In a step S108, an avatar in relation to the patient (also denoted as patient-specific avatar) is obtained as the result of the linking S106.

The method 100 still further comprises a step S110 of generating an interactive avatar of the patient by supplementing the obtained S108 avatar of the patient with a user interaction tool.

Optionally, the method 100 comprises a step S112 of outputting the generated S110 interactive avatar of the patient, in particular on a graphical user interface (GUI).

Alternatively or in addition, the method 100 may comprise a step S114 of receiving, by means of the interactive user tool, a request for medical data retrieval. Further alternatively or in addition, the method 100 may comprise a step S116 of outputting medical data, in particular according to a received S114 request. Still further alternatively or in addition, the method 100 may comprise a step S118 of receiving at least one further medical knowledge dataset in relation to the patient for updating the interactive avatar of the patient.

The method 100 may be used (in particular for optical guidance) during an interventional procedure and/or during a consultation.

Fig. 2 schematically illustrates an exemplary architecture of a computing device for generating an interactive avatar of a patient for medical data retrieval. The computing device is generally referred to by the reference sign 200.

The computing device 200 comprises a first interface 202 configured for receiving a graphical representation of a human body. The graphical representation of the human body may be patient-specific or non-patient-specific.

The computing device 200 further comprises a second interface 204 configured for receiving at least one (e.g., textual and/or image) medical knowledge dataset in relation to the patient from a medical knowledge database.

The computing device 200 further comprises a linking module 206 configured for executing an anatomical location algorithm for linking (e.g., textual and/or image) medical information comprised in the received at least one (e.g., textual and/or image) medical knowledge dataset to one or more associated locations of the received graphical representation. The linking module 206 may be further configured for obtaining an avatar of the patient.

The computing device 200 still further comprises a generating module 210 configured for generating an interactive avatar of the patient by supplementing the obtained avatar of the patient with a user interaction tool.

Optionally, the computing device 200 comprises a third interface 212 configured for outputting the generated interactive avatar of the patient, in particular on a GUI.

Alternatively or in addition, the computing device 200 may comprise a fourth interface (also denoted as medical data retrieval request module) 214 configured for receiving, by means of the interactive user tool, a request for medical data retrieval.

Alternatively or in addition, the computing device 200 may comprise a fifth interface 216 configured for outputting medical data, in particular according to a received request.

Alternatively or in addition, the computing device 200 may comprise a sixth interface 218 configured for receiving at least one further medical information dataset in relation to the patient for updating the interactive avatar of the patient.

Any one of the first interface 202, the second interface 204, and optionally the fourth interface 214 and/or the sixth interface 218, may be comprised in a common input interface 226. Alternatively or in addition, the optional third interface 212 and the optional fifth interface 216 may be comprised in a common output interface 227. Further alternatively or in addition, any one of the first interface 202, the second interface 204, and optionally the third interface 212, the fourth interface 214, the fifth interface 216 and the sixth interface 218 may be comprised in a common input-output interface 228.

The linking module 206 may comprise a (in particular patient-specific) avatar obtaining submodule 208. Alternatively or in addition, the computing device 200 may comprise the linking module 206 and a (in particular patient-specific) avatar obtaining module 208.

The computing device 200 may comprise a processor 230 (e.g., a central processing unit, CPU, and/or a graphics processing unit, GPU). Any one of the linking module 206 and the (in particular patient-specific) avatar obtaining module (or submodule) 208 may be comprised in the processor 230.

As displayed in Fig. 2, the computing device 200 may further comprise a memory 232.

The memory 232 may comprise a plurality of memory blocks. Alternatively or in addition, the memory 232 may be configured for storing a computer program for executing the method 100. Further alternatively or in addition, the memory 232 may be configured for storing the graphical representation, the medical knowledge database, and/or the (in particular interactive) avatar of the patient.

The computing device 200 may be used (in particular for optical guidance) during an interventional procedure and/or during a consultation.

The computing device 200 may be configured for performing the method 100.

A system (not shown) for generating an interactive avatar of a patient for medical data retrieval may comprise the computing device 200, a graphical representation database configured for storing a graphical representation of a human body, and a medical knowledge database configured for storing at least one medical knowledge dataset in relation to the patient.

The first interface 202 of the computing device 200 may be configured for receiving a graphical representation of a human body from the graphical representation database. Alternatively or in addition, the second interface 204 of the computing device 200 may be configured for receiving at least one (e.g., textual and/or) medical knowledge dataset in relation to the patient from the (e.g., textual and/or) medical knowledge database.

The inventive technique (e.g., comprising the method 100, the computing device 200, and/or the system comprising the computing device 200) may also be denoted as (e.g., generating and/or using a) bottom-up anatomy-data representation of a (in particular human) digital twin (and/or avatar). Alternatively or in addition, the (in particular interactive) avatar of the patient (also denoted as patient-specific avatar) obtained by the inventive technique may also be denoted as full-scale anatomically accurate bottom-up representation of a digital twin avatar.

By the inventive technique, the full-scale anatomically accurate bottom-up representation of a digital twin avatar of the human body is provided (and/or developed).

A digital twin (and/or, in particular interactive, patient-specific avatar) may be defined as complete human anatomy, 3D mesh corresponding to that anatomy, patient-specific clinical data corresponding to that anatomy and/or general medical knowledge corresponding to that anatomy. Alternatively or in addition the (e.g., patient-specific) digital twin may comprise a data object comprising the 3D mesh, the patient-specific clinical data and/or the general medical knowledge.

Alternatively or in addition the patient-specific avatar may comprise a graphical representation (e.g., of the digital twin) .

A digital twin (and/or, in particular interactive, patient-specific avatar) can be the fundamental basis for the providing (and/or development) of patient digital twinning as the digital twin (and/or patient-specific avatar) establishes a relationship between clinical entities, medical knowledge and the (e.g., human digital twin) avatar and its constituent canonical organs. The representation of a patient by the digital twin (and/or patient-specific avatar) can be useful is several applications such as visualization of the clinically enriched digital avatar of the patient for optical guidance, unique navigation interface (e.g., embodied by the user interaction tool) for browsing clinical data, patient education, and/or organization of clinical knowledge for systematic search.

By the inventive technique, an anatomically indexed patient data model of the human digital twin avatar (embodied by the, in particular interactive, patient-specific avatar) is provided as well as a procedure for linking and populating the patient data model with clinical data points such as imaging, reports, and/or wearable data.

The anatomically indexed patient data model may comprise a hierarchical organization (also denoted as hierarchical structure) of anatomical structures (also denoted as anatomical entities), in particular as defined by a medical ontology.

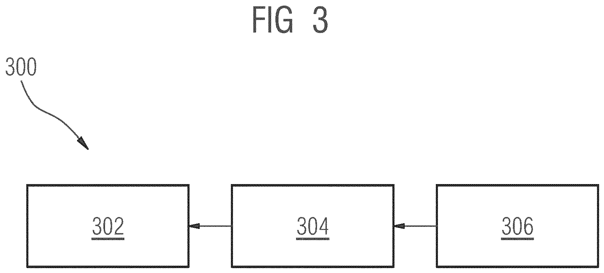

Fig. 3 schematically depicts an exemplary hierarchy of entity class relationships.

The (e.g., digital twin) avatar of the (e.g., class-specific, and/or patient-specific) human body may comprise (or may be comprised of) anatomical entity classes namely organs 306, organ systems 304 and the entire body 302, organized in a hierarchical manner 300, as schematically illustrated in Fig. 3. The organization of the anatomy in the (e.g., virtual) avatar may be informed by clinical ontologies such as the foundational model of anatomy (FMA) and/or the systematized nomenclature of medicine (SNOMED) (e.g., in relation to a, and/or per, body part).

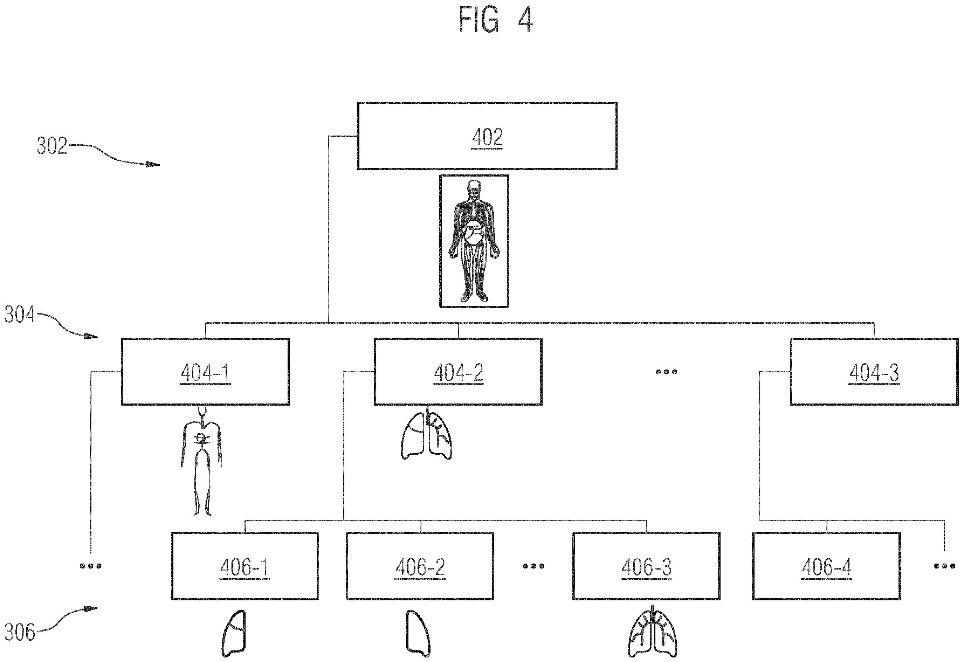

Fig. 4 schematically depicts an organization of anatomical structures (and/or anatomical entities) 302; 304; 306 in the (e.g., virtual) avatar 402, with examples of organ systems 304 and organs 306. The avatar 402 may also be denoted as human digital twin avatar.

The avatar 402 in Fig. 4 may be class-specific and/or patient-specific.

In the exemplary scheme of Fig. 4, at the top level of the hierarchical organization 300 is the 3D external representation (embodied by the full avatar) 402 of the entire human body 302, which may include a canonical representation for each biological sex type (e.g., for a class-specific avatar, and/or a patient-specific avatar), and/or a personalized representation based on patient-specific inputs (e.g., for the patient-specific avatar).

The next level of the hierarchy 304, also denoted as children entities of the entire body, in the exemplary scheme of Fig. 4 comprises (or is comprised of) all the major organ systems of the body such as cardiovascular system 404-1, respiratory system 404-2, skeletal system, and/or any further organ system 404-3, e.g., represented by a mesh.

The organ systems 404-1; 404-2; 404-3 may be represented by a canonical mesh (e.g., for a class-specific avatar, and/or a patient-specific avatar) and/or patient-specific meshes (e.g., for the patient-specific avatar), in particular whenever a segmentation output of corresponding medical imaging data is available.

At the next level of the hierarchy 306 in Fig. 4, the organ systems 304 comprise (or are comprised) of individual organs (also denoted as children organs). For example, the respiratory system 404-2 may comprise (or may be compromised of) organs such as lung lobes 406-1, right and left lungs 406-2, and/or the airway tree 406-3.

In an alternative embodiment (not shown), the lung lobes may be arranged at a (e.g., the next) lower hierarchical level below the right and left lungs 406-2. Alternatively or in addition, according to the alternative embodiment, the lung lobes may correspond to children entities (or children organs) of the lung.

Further constituent organs (or at least one further constituent organ) 406-4 of the further organ system 404-3 are also indicated schematically in Fig. 4.

The organs 406-1; 406-2; 406-3; 406-4 in turn may be represented by canonical meshes (e.g., for a class-specific avatar, and/or a patient-specific avatar) and/or personalized meshes (e.g., for the patient-specific avatar), in particular whenever a segmentation of the patient medical imaging data is available.

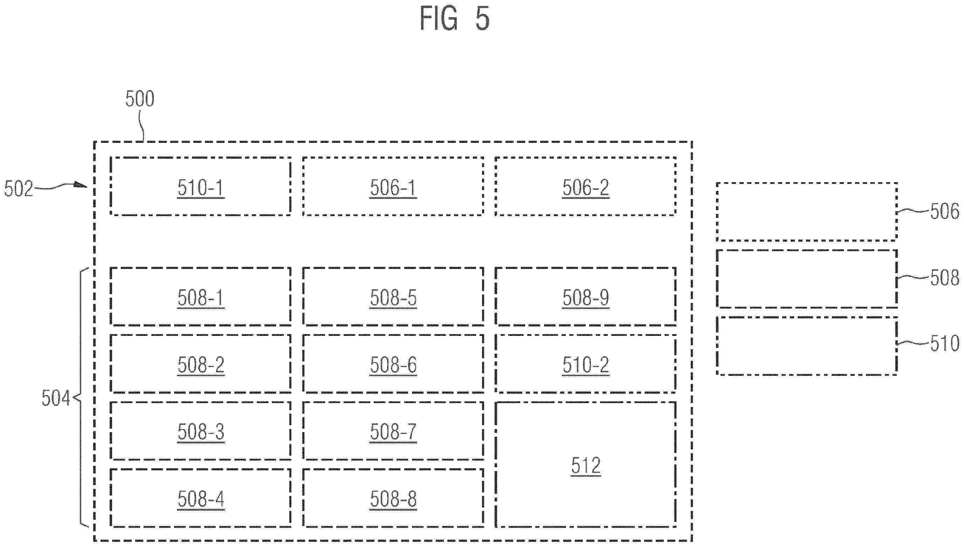

The anatomically indexed patient data model (e.g., embodied by the avatar 402) may further comprise anatomical indexing. Each anatomical structure (and/or anatomical entity class, briefly also: anatomical entity) may be associated with a data object 500 as schematically illustrated in Fig. 5.

The constituents of the data object 500 may be briefly denoted as (in particular non-patient-specific) medical knowledge 506 (indicated by dotted lines in Fig. 5), (in particular patient-specific) non-imaging data 508 (indicated by dashed lines in Fig. 5) and (in particular patient-specific) imaging data 510 (indicated by dashed-double-dotted lines in Fig. 5).

Any one of the constituents of the data object 500 may be denoted as data point and/or data set.

At reference sign 502 in Fig. 5, common data (e.g., per class of human bodies, also denoted as class members) are depicted.

Each class of human bodies (also denoted as anatomical class) has data members that represent the physical aspect of the entity in the form of a 3D mesh, medical knowledge associated with the entity from medical textbooks, journals or guidelines, or non-imaging clinical data.

The data common to all the instances of the class of human bodies (and/or anatomical class) in the schematic illustration of Fig. 5 exemplarily indicates a canonical 3D mesh 510-1, medical knowledge 506-1 and ontology concepts 506-2.

At reference sign 504 in Fig. 5, patient-specific data (also denoted as data of instance members, data specific to an instance of a class, and/or patient's clinical data) are schematically illustrated.

As the patient's clinical data 504 in Fig. 5, exemplarily the non-imaging data 508 of clinical notes 508-1, diagnosis reports 508-2, (e.g., prescribed) medications 508-3, laboratory data (briefly: lab data) 508-4, diagnoses 508-5 (e.g., to be included in a diagnosis report 508-2), (e.g., previous, and/or planned) procedures (also denoted as surgeries) 508-6, vital signs 508-7, (e.g., previous, and/or up-to-date) immunizations 508-8, and wearable data 508-9 (e.g., comprising continuous measurements of the vital signs 508-7) are shown.

The patient's clinical data 504 in Fig. 5, further exemplarily comprise a personalized segmentation 510-2 as imaging data 510.

As schematically shown in Fig. 5 at reference sign 512, data objects of the children anatomical entities may be comprised in the data object 500.

Children anatomical entities (and/or children anatomical structures) according to the hierarchical organization 300 as exemplified in Fig. 3 may comprise the anatomical entities (and/or anatomical structures) at a lower hierarchical level. E.g., in Fig. 4 the children anatomical entities (and/or children anatomical structures) of the respiratory system 404-2 may comprise the lobe 406-1, lungs 406-2 and airway tree 406-3.

Alternatively or in addition, the data object 500 exemplified in Fig. 5 may relate to an anatomical structure (e.g., the cardiovascular system 404-1 of Fig. 4) at one hierarchical level, and the data objects of the children anatomical entities may comprise related data objects at a lower hierarchical level (e.g., the heart, not shown).

The inventive technique (e.g., comprising the method 100, and/or the computing device 200) may comprise one or more algorithms (also denoted as models) to index clinical data objects with anatomical entities (e.g., comprising anatomic location algorithm).

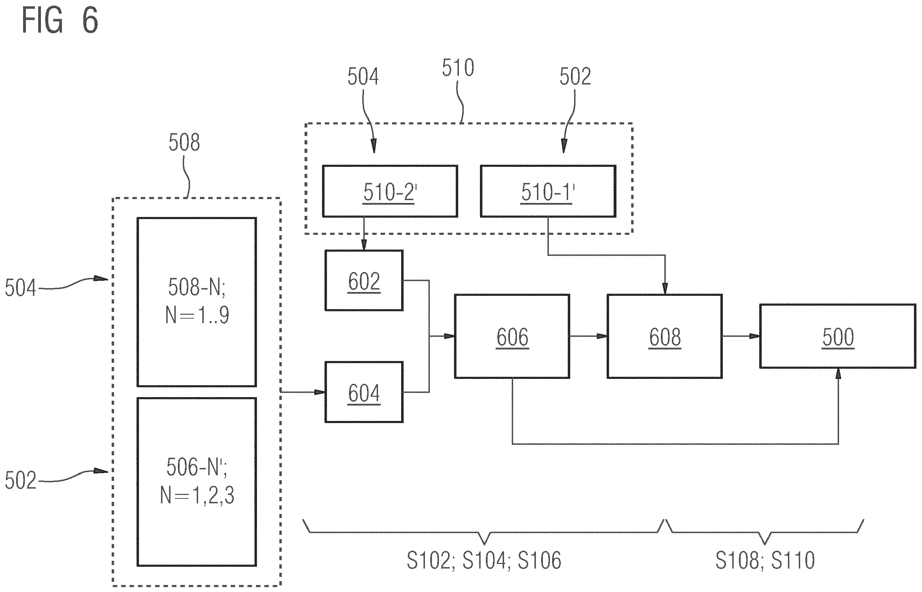

Fig. 6 schematically illustrates the extraction of data from imaging and non-imaging data (also denoted as inputs) using a variety of algorithms.

For a given datatype, in the example of Fig. 6, artificial intelligence (AI) algorithms are used to link the clinical data point to one or more corresponding anatomical structures (also denoted as anatomical entities), which are connected to an ontological knowledge graph (also denoted as graph data structure). The ontological knowledge graph (and/or graph data structure) maintains the relationship between the clinical data point, the clinical concept and the anatomical location corresponding to the concept in a human digital twin (and/or avatar). The algorithms used to link data points depend on the type of clinical data.

The non-imaging data in Fig. 6 are collectively shown at reference sign 508. The imaging data in Fig. 6 are collectively shown at reference sign 510. Each of the non-imaging data 508 and the imaging data 510 comprises patient-specific data 504 and common knowledge 502.

For the non-imaging data 508 such as a patient's clinical data points 508-N (N=1..9) (e.g., received in the step S104) or medical knowledge resources (also denoted as medical information sources, e.g., received in the step S104) such as medical textbooks (and/or dictionaries) 506-1', journal and/or conference publications 506-2', and guidelines 506-3', digital technology information (DTI) natural language processing (NLP) algorithms 604 such as RadBERT (bidirectional encoder representations from transformers adapted for radiology) are used to extract clinical entities (and/or anatomical structures) from the text. These clinical entities may, at reference sign 606, be matched to concepts in one of the relevant clinical ontologies (e.g., received in the step S104 of the method 100) such as SNOMED or RADLex (a digital radiology lexicon) using a text matching algorithm such as the python package BM25. As a result, a non-imaging clinical data structure 508 is linked to a concept identity (ID) of a clinical entity.

A clinical entity may be, or may comprise, a specific term in clinical text data that represents a medical concept, such as a symptom, disease, procedure, laboratory result (briefly also denoted as lab), imaging, and/or (e.g., medical) history. Alternatively or in addition, a clinical entity may be formulated in layman language.

Alternatively or in addition, a clinical concept may correspond to, or may comprise, a clinical idea to which a unique concept identifier has been assigned in a medical ontology database. Alternatively or in addition, a clinical concept may be formulated in expert language, and/or according to a predetermined structure.

Further alternatively or in addition, a clinical concept ID may correspond to, or may comprise, a unique identifier representing the clinical concept.

An example of an NLP algorithm for clinical entity extraction is, e.g., provided by R. Ghosh et al. in the preprint arXiv:2306.02492 ("RadLing: Towards Efficient Radiology Report Understand"), the disclosure of which is herein included by reference.

Alternatively or in addition, image processing algorithms are, e.g., provided by F.C. Ghesu et al. in IEEE Trans Pattern Anal Mach Intell 2019;41(1):176-189. 16 ("Multi-scale deep reinforcement learning for real-time 3D-landmark detection in CT scans") and Yang et al., Medical Image Computing and Computer Assisted Intervention- MICCAI 2017: 20th International Conference, Quebec City, QC, Canada, September 11-13, 2017, Proceedings, Part III 20. Springer International Publishing, 2017 ("Automatic liver segmentation using an adversarial image-to-image network"), the disclosures of both of which are herein included by reference.

For imaging data 510 such as (in particular patient-specific 504) medical imaging studies from devices and/or a picture archiving and communication system (PACS) 510-2', DTI image processing algorithms 602 may be used to segment the relevant anatomies, morphologies and findings from the image. The output labels of these results may be mapped to a clinical concept in the ontology, as indicated at reference sign 606, using a text matching algorithm. As a result, an imaging data structure 510-2' is linked to a concept ID of a clinical entity.

The clinical concept 606 (e.g., obtained from non-imaging data 508, imaging data 510, or both) is linked to one or more relevant anatomical structures (and/or anatomical entities) using a graph search (e.g., within a graph data structure and/or an ontological knowledge graph). Clinical ontologies 606 may be represented in graph data structures where each node represents a clinical concept, and the edges between the nodes represent various ontological relationships such as hierarchy, disease type, anatomical location, and/or lab values. The graph search may start at the node representing the clinical concept and iteratively traverse the graph, along edges that indicate anatomical relationships until the location corresponding to the concept is reached. For example, for an input clinical report (and/or diagnosis report 508-2) which consists of a sentence, "patient suffers with chronic cough", the NLP algorithm 604 may extract the clinical entity "cough". This entity may be matched at reference sign 606 to a SNOMED ontology concept "Cough (finding) SCTID: 49727002". Then, at reference sign 608 a graph search may be used to identify the closest anatomical entity to cough in the SNOMED ontology, e.g., to identify "lungs". The canonical mesh for lungs may be used to represent this clinical report. If imaging for the lungs is available for this patient, the segmentations 510-2' of anatomies and findings from the imaging 510-2' may be used to update the personalized anatomical entity meshes of the data object 500. For publicly available imaging databases 510-1', such as the Anatomography BodyPart3D, the meshes may be directly mapped to an anatomical ontology 608 such as FMA and/or SNOMED.

The publicly available imaging data 510-1', the medical information sources 506-1'; 506-2'; 506-3', may be received once, e.g., in the steps S102 and S104 of the method 100, respectively, and stored (e.g., in the memory 232 of the computing device 200, and/or in a computing cloud) for later use for a variety of patients.

The inventive technique serves to represent a (e.g., interactive, and/or digital twin) avatar of the patient (and/or, in particular individual, human body) comprehensively, including the constituent anatomical structures (and/or anatomical entities) and the fully enriched data associated with these structures (and/or entities). The use cases for this anatomically indexed patient data model are wide-ranging.

According to a first use case of the inventive technique, a multi-search of clinical data may be performed. Since each data point is linked to its corresponding anatomical structure (and/or anatomical entity), it can assist in a multi-search of a patient's clinical history. For instance, it can be used to pull up all the data relevant to a patient's liver including lab values, reports, imaging, and/or pathology since they will all be indexed by the anatomical structure (and/or anatomical entity) liver.

According to a second use case of the inventive technique, which is combinable with the first use case, a novel navigation of clinical data is provided. The full-scale representation of a patient's (e.g., digital twin) avatar presents a unique opportunity to enable a user (e.g., a medical practitioner and/or the patient) to browse the avatar as one would browse a geographical navigation system. Each data member is the data object 500, as exemplified in Fig. 5, may be represented as an individual data layer (e.g., according to the hierarchical organization of Figs. 3 or 4) of the avatar. The user may choose a particular layer to see data corresponding to that layer. The user may zoom in and out of the avatar to see anatomical structures (and/or anatomical entities) present in the body. The user may toggle on and off different anatomical structures that are visible to the user to adjust the view. Once the user selects an anatomical structure (and/or anatomical entity) in a specific data layer, the data corresponding to that that anatomical structure (also denoted as anatomy and type) may be displayed. For example, if the user selects the liver in the "laboratory values" layer, the liver function tests of the patient may be displayed.

According to a third use case, which is combinable with the first use case and/or the second use case, a conversion of medical reports into infographics may be provided. Since a full-scale representation of the patient data from clinical reports to corresponding 3D meshes is available with the inventive technique, it is possible to generate on-demand automated infographics to convert complex and cumbersome clinical reports and other data to easily digestible diagrams that are easy to understand. Applications such as these can be used during consultations, treatments, and/or for patient education and engagement.

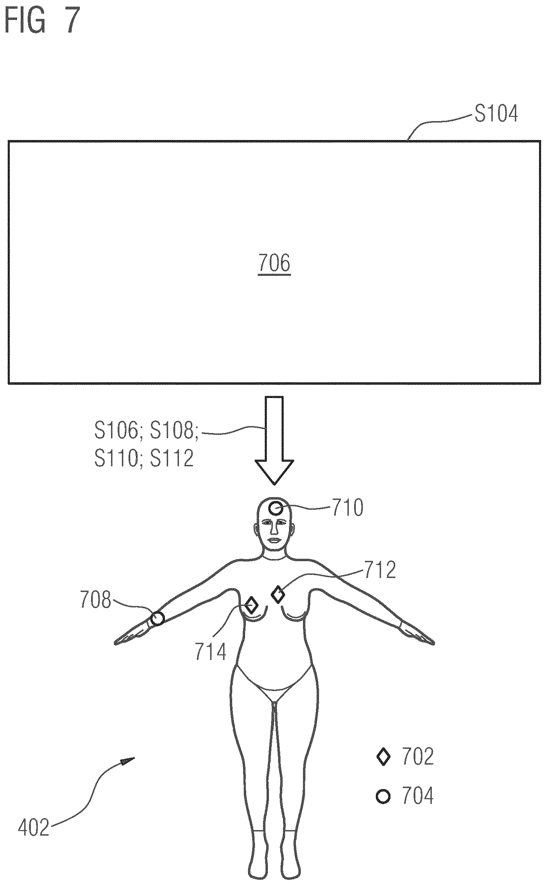

Fig. 7 shows a schematic example of generating an avatar 402 providing an infographic of a clinical note from a physician.

In the step S104 of the method 100, as indicated at reference sign 706 the exemplary collection of clinical notes (embodying textual medical information in one or more datasets) is received: "76-year-old women with memory problems, dizziness, who recently dropped a glass from the right hand. History of chemoradiation and/or breast cancer, atrial fibrillation and coronary artery disease (CAD). Right hand weakness and abnormal neuro exam with some right sided weakness. Concern for stroke, dementia, and metastatic disease."

Based on the method steps S106; S108; S110; S112, the avatar 402 may be output with indicated locations of the patient history 702 and indicated locations of the patient's current complaint 704. At reference sign 708, the location of the recently dropped glass is indicated. At reference sign 710, the location of the current memory problems, and/or dizziness is indicated. At reference sign 712, the location of the historic information of CAD, and/or atrial fibrillation is indicated. At reference sign 714, the location of historic information of breast cancer, and/or chemoradiation is indicated.

Wherever not already described explicitly, individual embodiments, or their individual aspects and features, described in relation to the drawings can be combined or exchanged with one another without limiting or widening the scope of the described invention, whenever such a combination or exchange is meaningful and in the sense of this invention. Advantages which are described with respect to a particular embodiment of present invention or with respect to a particular figure are, wherever applicable, also advantages of other embodiments of the present invention.

### List of Reference Signs

- 100: Method
- S102: Step of receiving a graphical representation
- S104: Step of receiving one or more medical knowledge datasets
- S106: Step of linking medical information to one or more locations of the graphical representation
- S108: Step of obtaining a patient-specific avatar
- S110: Step of generating an interactive patient-specific avatar
- S112: Step of outputting the interactive patient-specific avatar
- S114: Step of receiving a request for (in particular patient-specific) medical data retrieval
- S116: Step of outputting (in particular patient-specific) medical data
- S118: Step of receiving one or more further medical knowledge datasets
- 200: Computing device
- 202: 1^{st} interface
- 204: 2^{nd} interface
- 206: Linking module
- 208: Patient-specific avatar obtaining (sub)module
- 210: Generating module
- 212: 3^{rd} interface
- 214: 4^{th} interface
- 216: 5^{th} interface
- 218: 6^{th} interface
- 226: Input interface
- 227: Output interface
- 228: Input-output interface
- 230: Processor
- 232: Memory
- 300: Hierarchical organisation of anatomical structures
- 302: Entire body
- 304: Organ system
- 306: Organ
- 402: Avatar
- 404-1: Cardiovascular system
- 404-2: Respiratory system
- 404-3: Organ system, e.g., represented by a canonical mesh
- 406-1: Lung lobes
- 406-2: Lung
- 406-3: Airway Tree
- 406-4: Constituent Organ(s)
- 500: Data object of an anatomical identity
- 502: Common data (e.g., per class of human bodies)
- 504: Patient-specific data
- 506: Medical knowledge
- 506-1: Medical information
- 506-1': Medical textbooks, and/or dictionaries
- 506-2: Ontology concepts
- 506-2': Journals, and/or conference publications
- 506-3': Guidelines
- 508: Non-imaging data
- 508-1: Clinical notes
- 508-2: Diagnosis reports
- 508-3: Medications
- 508-4: Laboratory Data
- 508-5: Diagnoses
- 508-6: Procedures
- 508-7: Vital signs
- 508-8: Immunizations
- 508-9: Wearable data
- 510: Imaging data
- 510-1: Canonical 3D mesh
- 510-1': Imaging data acquired from public databases, e.g., BodyPart3D
- 510-2: Personalized segmentation
- 510-2': Imaging data acquired by a scanner, and/or stored in PACS
- 512: Data object of the children anatomical entities
- 602: (e.g., DTI) Image processing algorithm
- 604: (e.g., DTI) Natural language processing (NLP) algorithm
- 606: Clinical concept(s) in ontologies
- 608: Anatomical ontologies
- 702: Patient history
- 704: Patient's current complaint
- 706: Textual medical information about the patient
- 708: (E.g., recent) Location indication of dropped glass
- 710: (E.g., current) Location information of memory problems, and/or dizziness
- 712: (E.g., historic) Location information of CAD, and/or atrial fibrillation
- 714: (E.g., historic) Location information of breast cancer, and/or chemoradiation

## Claims

1. Method (100) for generating an interactive avatar of a patient for medical data retrieval, comprising the method steps of:
- Receiving (S102) a graphical representation of a human body;
- Receiving (S104) at least one medical knowledge dataset in relation to the patient from a medical knowledge database;
- Executing an anatomical location algorithm for linking (S106) medical information comprised in the received (S104) at least one medical knowledge dataset to one or more associated locations of the received (S102) graphical representation of the human body for obtaining (S108) an avatar of the patient; and
- Generating (S110) an interactive avatar of the patient by supplementing the obtained (S108) avatar of the patient with a user interaction tool.

2. Method (100) according to claim 1, further comprising the step of:
- Outputting (S112) the generated (S110) interactive avatar of the patient, in particular on a graphical user interface, GUI.

3. Method (100) according to any of the preceding claims,
wherein the graphical representation comprises a, in particular three-dimensional, 3D, mesh representation; and/or
wherein the graphical representation is algorithmically generated.

4. Method (100) according to any of the preceding claims, wherein the graphical representation, the at least one medical knowledge dataset, , and/or the, in particular interactive, avatar of the patient are stored in a local database, and/or are stored in a computing cloud; optionally
wherein the graphical representation and the at least one medical knowledge dataset are stored at different locations.

5. Method (100) according to any of the preceding claims, wherein the graphical representation comprises a biological sex type, and/or a development state, in particular comprising infant, adolescent and/or grown-up.

6. Method (100) according to any of the preceding claims, wherein the received (S104) at least one medical knowledge dataset comprises medical imaging data of the patient, in particular received from a medical scanner, optionally wherein the medical imaging data comprise annotations.

7. Method (100) according to any of the preceding claims, wherein the received (S104) at least one medical knowledge dataset comprises non-imaging medical data of the patient.

8. Method (100) according to any of the preceding claims, wherein the linking (S106) of textual medical information comprised in the received (S104) at least one medical knowledge dataset to the one or more associated locations of the received (S102) graphical representation is performed by means of natural language processing, NLP, and/or by means of a text matching algorithm to a medical ontology database.

9. Method (100) according to any of the preceding claims, wherein the linking (S106) of the medical information comprised in the received (S104) at least one medical knowledge dataset to the one or more associated locations of the received (S102) graphical is performed by means of an artificial intelligence, AI.

10. Method (100) according to any of the preceding claims, wherein the graphical representation and/or the medical information comprised in the received (S104) at least one medical knowledge dataset is/are hierarchically organized, in particular comprising the entire human body, organ systems, organs and suborganic structures as subsequent hierarchical layers.

11. Method (100) according to any of the preceding claims, wherein the medical information comprised in the received (S104) at least one medical knowledge dataset is represented by a graph data structure, wherein nodes of the graph data structure comprise clinical concepts, and wherein edges between nodes of the graph data structure comprise relationships between the clinical concepts.

12. Method (100) according to any of the preceding claims, further comprising at least one of the steps of:
- Receiving (S114), by means of the interactive user tool, a request for medical data retrieval;
- Outputting (S116) medical data, in particular according to a received (S114) request; and
- Receiving (S118) at least one further medical knowledge dataset in relation to the patient for updating the interactive avatar of the patient.

13. Use of the method (100) according to any of the preceding claims, in particular for optical guidance, during an interventional procedure and/or during a consultation.

14. Computing device (200) for generating an interactive avatar of a patient for medical data retrieval, comprising:
- A first interface (202) configured for receiving a graphical representation of a human body;
- A second interface (204) configured for receiving at least one medical knowledge dataset in relation to the patient from a medical knowledge database;
- A linking module (206) configured for executing an anatomical location algorithm for linking medical information comprised in the received at least one medical knowledge dataset to one or more associated locations of the received graphical representation of the human body
- for obtaining an avatar of the patient; and
- A generating module (210) configured for generating an interactive avatar of the patient by supplementing the obtained avatar of the patient with a user interaction tool.

15. Computing device (200) according to the directly preceding claim, further configured to perform any one of the method steps, and/or comprise any one of the features of claims 2 to 12.

16. System for generating an interactive avatar of a patient for medical data retrieval, comprising:
- A graphical representation database configured for storing a graphical representation of a human body;
- A medical knowledge database configured for storing at least one medical knowledge dataset in relation to the patient; and
- A computing device (200) according to claim 14 or 15, wherein the first interface (202) of the computing device (200) is configured for receiving a graphical representation of a human body from the graphical representation database, and wherein the second interface (204) of the computing device (200) is configured for receiving at least one medical knowledge dataset in relation to the patient from the medical knowledge database.

17. A computer program product comprising program elements which induce a computing device (200) to carry out the steps of the method for generating an interactive avatar of a patient for medical data retrieval according to one of the preceding method claims, when the program elements are loaded into a memory of the computing device (200).

## Amended claims

### Amended claims in accordance with Rule 137(2) EPC.

1. Method (100), performed by a computing device (200), for generating an interactive avatar (402) of a patient for medical data retrieval, comprising the method steps of:
- Receiving (S102), at a first interface (202), a graphical representation of a human body;
- Receiving (S104), at a second interface (204), at least one medical knowledge dataset in relation to the patient from a medical knowledge database;
- Executing, by a linking module (206), an anatomical location algorithm for linking (S106) medical information comprised in the received (S104) at least one medical knowledge dataset to one or more associated locations of the received (S102) graphical representation of the human body for obtaining (S108) an avatar (402) of the patient; and
- Generating (S110), by a generating module (210), an interactive avatar (402) of the patient by supplementing the obtained (S108) avatar (402) of the patient with a user interaction tool;
wherein the method (100) is used for optical guidance during an interventional procedure.

2. Method (100) according to claim 1, further comprising the step of:
- Outputting (S112) the generated (S110) interactive avatar (402) of the patient, in particular on a graphical user interface, GUI.

3. Method (100) according to any of the preceding claims,
wherein the graphical representation comprises a, in particular three-dimensional, 3D, mesh representation; and/or
wherein the graphical representation is algorithmically generated.

4. Method (100) according to any of the preceding claims, wherein the graphical representation, the at least one medical knowledge dataset, and/or the, in particular interactive, avatar (402) of the patient are stored in a local database, and/or are stored in a computing cloud; optionally
wherein the graphical representation and the at least one medical knowledge dataset are stored at different locations.

5. Method (100) according to any of the preceding claims, wherein the graphical representation comprises a biological sex type, and/or a development state, in particular comprising infant, adolescent and/or grown-up.

6. Method (100) according to any of the preceding claims, wherein the received (S104) at least one medical knowledge dataset comprises medical imaging data (510) of the patient, in particular received from a medical scanner, optionally wherein the medical imaging data (510) comprise annotations.

7. Method (100) according to any of the preceding claims, wherein the received (S104) at least one medical knowledge dataset comprises non-imaging medical data (508) of the patient.

8. Method (100) according to any of the preceding claims, wherein the linking (S106) of textual medical information (706) comprised in the received (S104) at least one medical knowledge dataset to the one or more associated locations of the received (S102) graphical representation is performed by means of natural language processing, NLP (604), and/or by means of a text matching algorithm to a medical ontology (608) database.

9. Method (100) according to any of the preceding claims, wherein the linking (S106) of the medical information comprised in the received (S104) at least one medical knowledge dataset to the one or more associated locations of the received (S102) graphical representation is performed by means of an artificial intelligence, AI.

10. Method (100) according to any of the preceding claims, wherein the graphical representation and/or the medical information comprised in the received (S104) at least one medical knowledge dataset is/are hierarchically organized (300), in particular comprising the entire human body (302), organ systems (304; 404-1; 404-2; 404-3), organs (306; 406-1; 406-2; 406-3; 406-4) and suborganic structures as subsequent hierarchical layers.

11. Method (100) according to any of the preceding claims, wherein the medical information comprised in the received (S104) at least one medical knowledge dataset is represented by a graph data structure, wherein nodes of the graph data structure comprise clinical concepts (606), and wherein edges between nodes of the graph data structure comprise relationships between the clinical concepts (606).

12. Method (100) according to any of the preceding claims, further comprising at least one of the steps of:
- Receiving (S114), by means of the interactive user tool, a request for medical data retrieval;
- Outputting (S116) medical data, in particular according to a received (S114) request; and
- Receiving (S118) at least one further medical knowledge dataset in relation to the patient for updating the interactive avatar (402) of the patient.

13. Use of the method (100) according to any of the preceding claims, in particular for optical guidance, during a consultation.

14. Computing device (200) for generating an interactive avatar (402) of a patient for medical data retrieval, comprising:
- A first interface (202) configured for receiving a graphical representation of a human body;
- A second interface (204) configured for receiving at least one medical knowledge dataset in relation to the patient from a medical knowledge database;
- A linking module (206) configured for executing an anatomical location algorithm for linking medical information comprised in the received at least one medical knowledge dataset to one or more associated locations of the received graphical representation of the human body for obtaining an avatar (402) of the patient; and
- A generating module (210) configured for generating an interactive avatar (402) of the patient by supplementing the obtained avatar (402) of the patient with a user interaction tool;
wherein the computing device (200) is usable for optical guidance during an interventional procedure.

15. Computing device (200) according to the directly preceding claim, further configured to perform any one of the method steps, and/or comprise any one of the features of claims 2 to 13.

16. System for generating an interactive avatar (402) of a patient for medical data retrieval, comprising:
- A graphical representation database configured for storing a graphical representation of a human body;
- A medical knowledge database configured for storing at least one medical knowledge dataset in relation to the patient; and
- A computing device (200) according to claim 14 or 15, wherein the first interface (202) of the computing device (200) is configured for receiving a graphical representation of a human body from the graphical representation database, and wherein the second interface (204) of the computing device (200) is configured for receiving at least one medical knowledge dataset in relation to the patient from the medical knowledge database.

17. A computer program product comprising program elements which induce a computing device (200) to carry out the steps of the method for generating an interactive avatar (402) of a patient for medical data retrieval according to one of the preceding method claims, when the program elements are loaded into a memory (232) of the computing device (200).
